# EUROPEAN PATENT SPECIFICATION

(11) **EP 0 672 669 B1**
(45) Date of publication and mention of the grant of the patent: **10.09.1997**
(21) Application number: 95201084.1
(22) Date of filing: 07.06.1993
(51) Int. Cl.: C07D 503/02

(54) **Process for the preparation of potassium clavulanate**
Verfahren zur Herstellung von Kalium Clavulanat
Procédé de préparation de clavualnate de potassium

(30) Priority: 11.06.1992 GB 9212379; 31.10.1992 GB 9222841; 14.12.1992 GB 9226061; 17.12.1992 GB 9226282
(43) Date of publication of application: 20.09.1995
(62) Divisional of application: 93913311.2
(73) Proprietor: SMITHKLINE BEECHAM PLC, Brentford, Middlesex TW8 9EP (GB)
(72) Inventor: Cook,Michael Allen, Worthing, West Sussex Bn14 8QH (GB); Wilkins,Robert Bennett, Irvine, Ayrshire KA11 5AP (GB)
(74) Representative: Vossius, Tilman

(56) References cited:
- EP-A- 0 026 044
- EP-A- 0 387 178
- EP-A- 0 594 099
- BE-A- 862 211
- GB-A- 1 508 977
- GB-A- 1 578 739
- GB-A- 2 264 944
- PL-A- 139 811
- Nomenclature of Organic Chemistry, Sections A,B,C,D,E,F and H, 1979 Edition, J. Rigaudy, S.P Klesney, Pergamon Press, pages 6,7
- Experimental report filed with reply of 14.07.1982 during examination of EP-A-0 026 044

## Description

This invention relates to a novel process for the preparation of clavulanic acid (I): 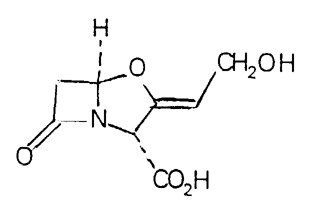 and pharmaceutically acceptable salts and esters thereof.

Clavulanic acid is normally prepared by the fermentation of a microorganism which produces clavulanic acid, such as various microorganisms belonging to various *Streptomyces* strains such as *S*. *clavuligerus* NRRL 3585, S. *jumoninensis NRRL* 5741, *S. katsurahamanus* IFO 13716 and *Streptomyces* sp. P 6621 FERM P2804 e.g. as described in JP Kokai 80-162993. The resulting aqueous broth may be subjected to conventional purification and concentration processes, for example involving filtration and chromatographic purification, such as disclosed in GB 1508977 and JP Kokai 80-62993, before extraction of the aqueous solution with an organic solvent to yield a solution of crude clavulanic acid in the organic solvent.

GB 1508977 discloses inter alia that salts of clavulanic acid can be obtained by absorbing the clavulanate anion in filtered broth on to an anion exchange resin, eluting therefrom with an electrolyte, desalting the resulting solution, applying the desalted solution to a further anion exchange resin, chromatographically eluting therefrom with an electrolyte, desalting the resulting solution and thereafter removing the solvent. This process can be used to give acceptable yields of pure material but the use of resin columns involves significant investment and they can introduce limitations in large scale production operations. It would therefore be desirable to have an alternative procedure available that involved few resin utilizing stages.

GB-A-1 578 739 describes *inter alia* various amine salts of clavulanic acid. GB-A-2.264944 and EP-A-0594099 are co-pending applications and describe the use of t-octylamine salts of clavulanic acid as intermediates in preparing potassium clavulanate.

GB 1543563 discloses a process for the preparation of clavulanic acid salts via precipitation of lithium clavulanate. GB 1578739 describes various amine salts of clavulanic acid as pharmaceutical compounds. EP 0026044 discloses the use of the tertiary-butylamine salt of clavulanic acid as a useful intermediate in the preparation of clavulanic acid. The salt has been disclosed in BE 862211, but only as a suitable ingredient for pharmaceutical formulations. PT.94.908 describes the use of tri-(lower alkyl)amine salts and the dimethylaniline salts of clavulanic acid in a purification process for clavulanic acid in which the triethylamine salt of clavulanic acid is formed and is then converted into a silyl diester of clavulanic acid. EP 03887178A discloses a process for the purification of clavulanic acid in which organic amines may be used to form an intermediate amine salt with clavulanic acid in an impure solution.

The present invention provides the use of the salt of clavulanic acid with t-octylamine as an intermediate in a process for the preparation of potassium clavulanate.

In a further aspect the present invention provides a process for the preparation and/or purification of potassium clavulanate which process comprises
i)
   a) obtaining impure clavulanic acid in a broth resulting from fermentation of a clavulanic acid - producing microorganism
   b) at least removing some of the suspended solids in the broth by filtration;
   c) extructing an acidified solution of the impure clavulanic acid obtained in step b) into an organic solvent
   d) contacting the extract with t-octylamine
ii) isolating the tertiary-octylamine salt of clavulanic acid formed;
iii) converting the thus formed salt into potassium clavulanate characterised in that the broth is acidified prior to solvent extraction.

In the process of the present invention the salt of clavulanic acid with the t-octylamine may be used to purify impure clavulanic acid during its preparation. Therefore the salt is be formed in a solution of clavulanic acid containing impurities, isolating the salt as a separate phase, eg as a solid precipitate, from the solution containing residual impurities, then reforming clavulanic acid or forming a potassium salt thereof.

Conveniently the t-octylamine itself is contacted with impure clavulanic acid itself in solution in an organic solvent.

The above process is suitably carried out in an organic solvent, which although preferably substantially dry, for example containing less than 6 g/L, eg 0.25-0.6 g/L of water, may contain some water, as a solvent for the clavulanic acid and the t-octylamine. A suitable degree of dryness may be achieved by conventional dewatering processes such as centrifuging. Water present in the solvent may be dissolved or in the form of droplets of a separate phase.

The solution of clavulanic acid in organic solvent may be obtained by extraction of an acidified aqueous solution of clavulanic acid such as the fermentation liquor referred to above. If the initial source of the clavulanic acid is a broth resulting from fermentation of clavulanic acid-producing microorganism. such as those mentioned above, then to obtain a solvent extract of a suitable concentration of clavulanic acid for use in this process it may be desirable not to extract the broth itself, but to at least remove some of the suspended solids in the broth, e.g by filtration prior to extraction. It may also be desirable in addition to pre-concentrate the aqueous solution of clavulanic acid obtained in fermentation, so that for example the aqueous solution of clavulanic acid is several times more concentrated in clavulanic acid than the starting broth, for example pre-concentrated to a concentration of ca. 10 - 100 mg/ml. e.g 10 - 40 mg/ml, such as 10 - 25 g/L clavulanic acid.

Suitable pre-concentration processes include absorption of the clavulanic acid onto an anion exchange resin, followed by elution of the clavulanic acid therefrom with an aqueous solution of an electrolyte such as sodium chloride, and optionally de-salting. It is also preferred to acidify the aqueous solution, e.g the broth or the pre-concentrated aqueous solution prior to solvent extraction, e.g to pH 1 to 3, e.g around pH 1.5 to 2.5. It is also preferred to dry or de-water the organic solvent extract prior to formation of the salt with the t-octylamine, e.g to less than 6g/L of water. Preferably the extraction is carried out at a temperature from 5 to 15°C.

Suitable organic solvents in which impure clavulanic acid may be contacted with the t-octylamine include hydrocarbon solvents such as toluene and hexane, ethers such as tetrahydrofuran, dioxan, diethyl ether, halogenated solvents such as dichloromethane and chloroform, ketones such as acetone and methyl isobutyl ketone, and esters such as ethyl acetate. Solvents which include a carbonyl group, eg those of the formula (III): 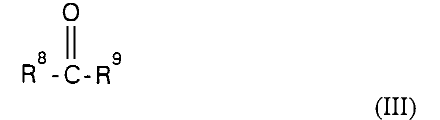 wherein R⁸ is a C₁₋₆ alkyl group or a C₁₋₆ alkoxy group and R⁹ is a C₁₋₆ alkyl group are examples of a sub-class of suitable solvents, for example organic ketones or organic alkanoate esters. The present invention also encompasses the use of mixtures of such solvents.

More suitably the organic solvent is one which can be used directly to extract the acidified aqueous for example organic alkyl alkanoate esters, ketones and certain aliphatic alcohols, or mixtures thereof, such as ethyl acetate, methyl acetate, propyl acetate, n-butyl acetate, methyl ethyl ketone, methyl isobutyl ketone, tetrahydrofuran, butanol and mixtures of such solvents. Of these the most suitable appear to be methyl isobutyl ketone, methyl ethyl ketone, and ethyl acetate. Suitable solvent mixtures include methyl ethyl ketone/methyl isobutyl ketone and tetrahydrofuran/methyl isobutyl ketone. A preferred solvent is ethyl acetate.

Suitable solvents for t-octylamine include those referred to above in which the clavulanic acid may be dissolved, or extracted, for example acetone, ethyl acetate, methyl isobutyl ketone, and methyl ethyl ketone.

It appears to be particularly desirable to include ketones such as acetone in the solvent system, as these appear to inhibit the formation of the salt of clavulanic acid with t-octylamine as an oil.

In general one equivalent of t-octylamine or a slight excess thereof per mole of clavulanic acid is used to produce the salt of clavulanic acid. Solutions of clavulanic acid and t-octylamine may for example be mixed slowly with stirring and the mixture stirred for some time after addition is complete. The reaction between the clavulanic acid or its labile derivative is suitably carried out at a temperature below ambient, for example 0 to 15°C, eg 0 to 10°C, eg 0 to 5°C. A suitable concentration for the clavulanic acid or its labile derivtive in the solution is at least 1.0 g/L, for example in the range 1.0 to 4.0 g/L of clavulanic acid. It may be advantageous to further concentrate the solvent extract to a concentration in excess of this eg greater than 20g/L.

For example in another procedure t-octylamine may be introduced by mixing it into a stream of a solution of the clavulanic acid in the solvent, so that the salt is formed in the stream, either in solution or as particles or suspended droplets of the dissolved salt in suspension. The t-octylamine introduced in this way may be introduced neat, or may be introduced as a solution in a solvent, for example the same organic solvent as the clavulanic acid is dissolved in.

The desired salt of clavulanic acid with t-octylamine may then be isolated. In this way, the salt of clavulanic acid with t-octylamine is separated from most or all of the impurities. Isolation may be effected in a conventional manner, for example by centrifugation or filtration.

In an alternative isolation procedure the salt of clavulanic acid with t-octylamine may be isolated from the organic solvent, if the solvent is wholly or partly immiscible with water, by contacting the solvent with water so as to extract the salt, which may be in solution or suspension, from the organic solvent and to form an aqueous solution of the salt. As salts of clavulanic acid with t-octylamine are fairly soluble in water, such an aqueous solution may be very concentrated, eg around 20-30% w:w.

In this way the bulk of organic impurities in the organic solvent solution of clavulanic acid remain in the organic solvent whilst the clavulanic acid, in the form of its salt with t-octylamine, in a relatively pure state is obtained in the aqueous solution. The aqueous solution of the clavulanic acid salt so formed may be subjected to conventional further treatment, eg purification, or conversion into clavulanic acid or a pharmaceutically acceptable salt or ester as described below.

In another alternative or additional procedure the clavulanic acid and t-octylamine may be mixed in solution in a first organic solvent, then the salt may be caused to separate from solution by addition of a second organic solvent. Suitably the first organic - solvent may be an organic ester such as ethyl acetate, and the second solvent may for example be a halogenated solvent such as chloroform, an ether such as diethyl ether, a hydrocarbon such as toluene, an alcohol such as ethanol, or a solvent of formula (III) above such as acetone or methyl isobutyl ketone.

In one embodiment of this invention, the salt of clavulanic acid with t-octylamine may be employed as an acetone soivate. Acetone soivates in some cases have advantageous stability and purity characteristics compared to salts of clavulanic acid with amines themselves. Such solvates are particularly useful in the present invention because they can often be isolated as a highly pure and stable crystalline compound.

Accordingly the present invention also provides the salt of clavulanic acid with t-octylamine in the form of an acetone solvate. During isolation and/or drying, some acetone may be lost since the strength of solvation may not be high, but the amount of acetone in the product is not critical.

The acetone solvate may be formed by contacting clavulanic acid with t-octylamine in the presence of acetone. In general, a solution containing clavulanic acid may be mixed with at least the same volume of acetone together with t-octylamine, when the salt is precipitated.

The t-octylamine may be dissolved in acetone and mixed with a solution of clavulanic acid in an organic solvent. Favoured organic solvents include ethyl acetate, tetrahydrofuran, methyl ethyl ketone, methyl isobutyl ketone and mixtures of such solvents, of which ethyl acetate is preferred. Alternatively an aqueous solution of the salt of clavulanic acid with t-octylamine, obtained by water extraction as outlined above, may be mixed with acetone to form the solvate. Suitably a concentrated aqueous solution of the salt may be mixed with excess acetone to form the solvate.

Recrystallisation of the salt of clavulanic acid or the acetone solvate may be advantageous to further reduce the level of impurities. A convenient solvent for the recystallisation is aqueous acetone. Such recrystallisation is performed in a conventional manner, for example the salt or solvate is dissolved in water, treated with a small amount of acetone, filtered, and then treated with larger volumes of acetone optionally with stirring and/or cooling to afford the recrystallised product.

The potassium clavulanate prepared by the processes of this invention include those described in GB 1508977 and 1508978 which are herein incorporated by reference.

The salt of clavulanic acid with t-octylamine optionally in the form of its acetone solvate may be converted into potassium clavulanate by for example ion-replacement in the case of the free acid or salts.

Ion-replacement may be performed using ion-exchange resins, for example by passing a solution of the salt through a bed of a cation exchange resin in potassium form. Suitable cation exchange resins include Amberlite IR 120 and equivalent resins.

Alternatively ion-replacement may be effected by reaction of the protonated amine cation with a salt-precusor compound, which may be a base, for example a carbonate, bicarbonate or hydroxide of a pharmaceutically acceptable alkali or alkaline earth metal, or a salt of an organic carboxylic acid with a pharmaceutically acceptable cation such as an alkali or alkaline earth metal, for example a salt of an alkanoic acid of formula (IV) :

R¹⁰-CO₂H (IV)

wherein R¹⁰ is an alkyl group, containing for example from 1 to 20 carbon atoms, preferably from 1 to 8 carbon atoms. Examples of suitable salts include the acetate, propionate or ethylhexanoate salts, potassium 2-ethylhexanoate being preferred. Typically the salt of clavulanic acid with t-octylamine in solution may be reacted with a salt of an alkali metal with acid (IV) in solution or suspension in a suitable solvent, which may for example be an organic solvent, water, or a mixture of water and an organic solvent such as isopropanol. Suitably solutions of the salt of clavulanic acid with t-octylamine and of the salt-precurssor compound may be mixed, and the pharmaceutically acceptable salt allowed to crystallise. Suitably the reaction may be carried out of a temperature below ambient, e.g. 0 to 15° C, e.g. 0 to 10°C, suitably 0 to 0.5°C. Suitably if the salt of clavulanic acid with t-octylamine is formed in aqueous solution it may be precipitated out by admixing the aqueous solution with excess acetone.

The invention will now be described by way of example only.

### Example 1

Filtered (RVF) *S. clavuligerus* fermentation broth containing 2 g/L at clavulanic acid was acidified to pH 1.6 with 25% v/v sulphuric acid and continuously extracted into ethyl acetate. The solvent extract was cooled to 3°C then dewatered by centrifuging, then dried with MgSO₄, then passed down a CPG carbon column. The carbon-treated extract was then concentrated by evaporation to a concentration of clavulanic acid of ca. 20 g/L, with a moisture content of ca. 0.06% v/v.

13.5ml of t-octylamine was mixed with 43 mls of fresh ethyl acetate. This mixture was slowly added to 400 ml of clavulanate rich ethyl acetate extract at a titre of 20 g/L clavulanic acid, with rapid stirring. The slurry was stirred for a further hour at 5°C. The t-octylamine clavulanate was subsequently isolated by filtration, and washed with ethyl acetate. Final drying was carried out overnight in a vacuum oven at 20°C, with a nitrogen bleed. Product weight = 12.44 g.

### Example 2

11g of the amine salt produced in example 1 was dissolved in 213 mls of isopropanol. The product did not dissolve readily, even after applying gentle warming, to 24°C. Water (3.75ml) was added to enable dissolution. Once a solution was obtained additional isopropanol (100ml) was added to reduce the water content prior to crystallisation. 30ml of 1.5 N. potassium 2-ethyl hexanoate in isopropanol was added over 15 minutes. The slurry was subsequently stirred for 1.5 hours at 5°C. The potassium clavulanate product was then filtered off. washed with small amounts of isopropanol then acetone, and dried under vacuum overnight, with a nitrogen bleed, at 20°C. Product weight = 7.29 g.

## Claims

1. A process for the preparation and/or purification potassium clavulanate which process comprises
i)
a) obtaining impure clavulanic acid in a broth resulting from fermentation of a clavulanic acid - producing microorganism
b) at least removing some of the suspended solids in the broth by filtration
c) extracting an acidified solution of the impure clavulanic acid obtained in step b) into an organic solvent; d) contacting the extract with t-octylamine;
ii) isolating the tertiary-octylamine salt of clavulanic acid formed;
iii) converting the thus formed salt into potassium clavulanate characterised in that the aqueous solution is acidified prior to solvent extraction.

2. A process according to claim 1 in which the aqueous solution of clavulanic acid is acidified to pH 1 to 3.

3. A process according to claim 2 in which the aqueous solution of clavulanic acid is acidified to pH 1.5 to 2.5.

## Patentansprüche

1. Verfahren zur Herstellung und/oder Reinigung von Kaliumclavulanat, wobei das Verfahren umfaßt
i)
a) Erhalten unreiner Clavulansäure in einer Brühe, die durch Fermentation eines Clavulansäure-produzierenden Mikroorganismus entsteht,
b) Entfernen mindestens einiger der suspendierten Feststoffe in der Brühe durch Filtration,
c) Extrahieren einer angesäuerten Lösung der in Schritt b) erhaltenen unreinen Clavulansäure in ein organisches Lösungsmittel,
d) Inkontaktbringen des Extrakts mit tert.-Octylamin;
ii) Isolieren des gebildeten tert.-Octylaminsalzes der Clavulansäure,
iü) Umwandeln des gebildeten Salzes in Kaliumclavulanat, dadurch gekennzeichnet, daß die wäßrige Lösung vor der Lösungsmittelextraktion angesäuert wird.

2. Verfahren nach Anspruch 1, wobei die wäßrige Lösung der Clavulansäure auf einen pH-Wert von 1 bis 3 angesäuert wird.

3. Verfahren nach Anspruch 2, wobei die wäßrige Lösung der Clavulansäure auf einen pH-Wert von 1,5 bis 2,5 angesäuert wird.

## Revendications

1. Procédé pour la préparation et/ou la purification de clavulanate de potassium, procédé qui comprend les étapes consistant
i)
a) à obtenir de l'acide clavulanique impur dans un bouillon résultant de la fermentation d'un micro-organisme producteur d'acide clavulanique,
b) à éliminer au moins certaines des matières solides en suspension dans le bouillon par filtration,
c) a extraire une solution acidifiée de l'acide clavulanique impur en solution dans un solvant organique obtenu à l'étape b),
d) à mettre en contact l'extrait avec de la tertio-octylamine ;
ii) à isoler le sel de tertio-octylamine d'acide clavulanique formé ;
iii) à transformer le sel ainsi formé en clavulanate de potassium, caractérisé en ce que la solution aqueuse est acidifiée avant l'extraction avec un solvant.

2. Procédé suivant la revendication 1, dans lequel la solution aqueuse d'acide clavulanique est acidifiée à un pH de 1 à 3.

3. Procédé suivant la revendication 2, dans lequel la solution aqueuse d'acide clavulanique est acidifiée à un pH de 1,5 à 2,5.
